Europäisches Patentamt

European Patent Office

Office européen des brevets

(11) Numéro de publication : **0 469 940 B1**

(12) **FASCICULE DE BREVET EUROPEEN**

(45) Date de publication du fascicule du brevet :
**02.02.94 Bulletin 94/05**

(51) Int. Cl.[5] : **C07C 15/107,** C07C 2/66,
C11D 1/22

(21) Numéro de dépôt : **91401831.2**

(22) Date de dépôt : **03.07.91**

(54) **Procédé de production de 2- et 3-phenylalcanes utilisant un catalyseur à base d'une mordénite particulière.**

(30) Priorité : **31.07.90 FR 9009878**

(43) Date de publication de la demande :
**05.02.92 Bulletin 92/06**

(45) Mention de la délivrance du brevet :
**02.02.94 Bulletin 94/05**

(84) Etats contractants désignés :
**AT BE DE ES GB IT NL**

(56) Documents cités :
**EP-A- 029 652
EP-A- 0 366 515
FR-A- 2 084 704
US-A- 4 731 497**

(73) Titulaire : **INSTITUT FRANCAIS DU PETROLE
4, avenue de Bois Préau
F-92502 Rueil-Malmaison (FR)**

(72) Inventeur : **Juguin, Bernard
Décédé (FR)**
Inventeur : **Joly, Jean-François
20 rue Béranger
F-75003 Paris (FR)**
Inventeur : **Raatz, Francis
25, rue de la Carrière
F-57500 Saint Avold (FR)**
Inventeur : **Martino, Germain
Bât. Condé, 80, avenue F. Lefebvre
F-78300 Poissy (FR)**
Inventeur : **Caullet, Philippe
4, rue Kellermann
F-68110 Illzach (FR)**

EP 0 469 940 B1

EP 0 469 940 B1

## Description

La présente invention concerne un procédé de production de 2-phénylalcanes et de 3-phénylalcanes par alkylation du benzène au moyen d'oléfine(s) linéaire(s) comprenant de 9 à 16 atomes de carbone, plus particulièrement de 10 à 14 atomes de carbone, en présence d'au moins un catalyseur à base d'une mordénite particulière.

Les 2- et 3-phénylalcanes obtenus selon l'invention constituent des composés pour la formulation, après sulfonation, de détergents biodégradables.

Actuellement, les bases pour détergents biodégradables font largement appel aux alkybenzènes linéaires. La production de ce type de composés est en croissance régulière : elle avoisine 650 000 tonnes/an à l'échelle de l'Europe de l'Ouest par exemple. Une des propriétés principales recherchée pour ces composés, après l'étape de sulfonation, est, outre leur pouvoir détergent, leur biodégradabilité. Pour assurer une biodégradabilité maximale, le groupement alkyl doit être linéaire et la distance entre le groupe sulfonate et le carbone terminal de la chaîne linéaire doit être maximal, le groupe phényl doit être le plus près possible d'une des extrémités du groupe alkyl. Les alkylbenzènes linéaires du type 2-et 3-phénylalcanes sont ainsi les plus appropriés pour atteindre cet objectif, les agents d'alkylation du benzène les plus intéressants étant constitués par les oléfines linéaires en $C_9$-$C_{16}$, de préférence $C_{10}$-$C_{14}$.

Les alkylbenzènes linéaires obtenus par alkylation du benzène au moyen d'oléfine(s) linéaire(s) sont préparés aujourd'hui par deux grands procédés.

Le premier procédé est licencié par UOP (B. Vora, P. Pujado, J. Spinner, T. Imai, Hydrocarbon Processing, Nov.1984, p.86 ; Petrochemical Handbook, Hydrocarbon Processing, Nov.1987, p.63). Ce procédé utilise lors de l'étape d'alkylation du benzène de l'acide fluorhydrique comme agent catalytique.

Le second procédé est licencié par ARCO Technology Inc (Petrochemical Handbook, Nov. 1985, p.127). Le catalyseur d'alkylation est ici de type Friedel-Craft, en particulier à base de Al-$C_{13}$.

Ces deux procédés présentent plusieurs inconvénients majeurs. Un des inconvénients résulte de la non-sélectivité des catalyseurs utilisés dans ces procédés. En effet, si ces catalyseurs sont très sélectifs du point de vue alkylation, ils conduisent à la formation de l'ensemble des phénylalcanes linéaires possibles. En plus de 2- et 3-phénylalcanes, il y a formation de 4-,5- et 6- phénylalcanes en raison de l'isomérisation de position de la double liaison de l'oléfine initiale.

Un autre inconvénient a trait à des contraintes d'environnement. Le procédé UOP basé sur l'utilisation d'acide fluorhydrique pose des problèmes de sécurité sévères d'une part et de retraitement des déchets d'autre part. Le procédé ARCO pose le problème classique des procédés basés sur des catalyseurs Friedel-Craft, en l'occurence le problème des rejets ; en effet, pour ce type de procédé, il est nécessaire de neutraliser les effluents par une solution basique en sortie de réacteur. A ces divers inconvénients s'ajoutent pour les deux procédés les difficultés liées à la séparation du catalyseur des produits de réaction.

Ces diverses contraintes expliquent l'intérêt qu'il y aurait à mettre au point un procédé d'alkylation du benzène par les oléfines linéaires en présence d'un catalyseur solide possédant des propriétés de sélectivité géométrique conduisant à une sélectivité améliorée en 2-phénylalcanes et 3-phénylalcanes. Une telle formule catalytique permettrait, d'une part, d'éliminer les problèmes d'environnement et de séparation catalyseur-effluents, et, d'autre part, de maximiser la production des 2-phénylalcanes et 3-phénylalcanes qui sont les produits les plus recherchés. De tels catalyseurs solides à sélectivité géométrique pourraient être constitués par certaines zéolithes. Ainsi, l'utilisation de ce type de solides pour l'alkylation du benzène a déjà été décrite à plusieurs reprises. Bowes (brevet US-A-3716596) a revendiqué l'utilisation d'une zéolithe H-ZSM4 de rapport molaire $SiO_2$/$Al_2O_3$ de 7,7 dès 1973. Plus récemment, Young (brevet US-A-4301317)a revendiqué toute une série de zéolithes pour l'alkylation du benzène par les oléfines linéaires longues. Parmi les structures citées par cet auteur, on trouve notamment : la cancrinite, la gmelinite, la mordénite, l'offrétite et la ZSM12. Avant utilisation, ces zéolithes doivent être transformées au moins partiellement en une forme hydrogène. La technique requise pour procéder à cette transformation consiste à remplacer les cations initialement présents dans le solide par des précurseurs de protons, tels que les ions ammonium, puis à calciner en présence de vapeur d'eau la forme ammonium ainsi obtenue. Le produit résultant de cette transformation initiale peut alors être soumis à divers traitements chimiques, en particulier des traitements de désalumination. Les zéolithes préparées selon cette technique (US-A-4301317) se révèlent assez performantes pour l'alkylation du benzène par les oléfines linéaires comprenant au moins 5 atomes de carbone. La température de réaction requise doit être supérieure à 50°C et la pression supérieure à 1 MPa.

D'autre part, le brevet US-A-4,731,497 décrit l'alkylation de benzène monoalkylé par des $\alpha$-oléfines à chaînes longues en présence d'un catalyseur comprenant une mordénite forme hydrogène.

Enfin, la demande de brevet EP-A-366.515 décrit un procédé d'alkylation du benzène par au moins une oléfine aliphatique en présence d'un catalyseur à base de mordénite désaluminée, caractérisé en ce que le

2

produit de la réaction est fractionné et que l'on fait réagir au moins une partie d'une fraction renfermant au moins un polyalkylbenzène avec du benzène au contact d'une mordénite désaluminée au cours d'une réaction de transalkylation.

La demanderesse a découvert que des mordénites préparées à partir des mordénites synthétisées en milieu fluorure selon le procédé de préparation décrit et revendiqué dans la demande de brevet français de numéro d'enregistrement national 89/13317 déposée le 10 octobre 1989 présentent des propriétés particulièrement intéressantes en alkylation du benzène au moyen d'oléfine(s) linéaire(s) comprenant de 9 à 16 atomes de carbone, par exemple de 10 à 14 atomes de carbone. Les catalyseurs préparés à partir de ces mordénites synthétisées en milieu fluorure (dites mordénites brutes de synthèse) présentent notamment des performances catalytiques supérieures pour la réaction pré-citée en vue de produire des 2- et 3-phénylalcanes à celles des catalyseurs préparés à partir de mordénites synthétisées en milieu classique OH-. Lesdites mordénites synthétisées en milieu fluorure (dites mordénites brutes de synthèse) correspondent de manière avantageuse aux mordénites décrites et revendiquées dans cette demande de brevet français 89/13317 dont une description partielle est incorporée ici à titre de référence.

La mordénite décrite et revendiquée dans ladite demande de brevet français a, après synthèse, la formule générale approchée $Na_2O,Al_2O_3,xSiO_2$ et est notamment caractérisée par :

a/ un nombre x compris entre 9 et 30, de préférence entre 9 et 20 (x étant le rapport molaire $SiO_2/Al_2O_3$),

b/ un diagramme de diffraction des rayons X représenté dans le tableau I de la description de la demande de brevet français 89/13317, ledit tableau I étant repris ci-dessous.

Cette mordénite brute de synthèse est également caractérisée par le fait qu'elle a été synthétisée en milieu fluorure.

L'identification des mordénites peut se faire de manière aisée à partir de leur diagramme de diffraction des rayons X. Ce diagramme de diffraction peut être obtenu à l'aide d'un diffractomètre en utilisant la méthode classique des poudres avec le rayonnement K alpha du cuivre. Un étalon interne permet de déterminer les valeurs des angles 2 thêta associées aux pics de diffraction. Les différentes distances interréticulaires $d_{hkl}$, caractéristiques de l'échantillon, sont calculées à partir de la relation de Bragg. L'estimation de l'erreur de mesure delta ($d_{hkl}$) se calcule en fonction de l'erreur absolue delta (2 thêta) affectée à la mesure de 2 thêta par la relation de Bragg. En présence d'un étalon interne, cette erreur est minimisée et prise couramment égale à ± 0,05°. L'intensité relative I/Io affectée à chaque valeur de $d_{hkl}$ est estimée à partir de la hauteur du pic de diffraction correspondant. Cette dernière peut également être déterminée à partir d'un cliché obtenu à l'aide d'une chambre de Debye-Scherrer. On utilise souvent une échelle de symboles pour caractériser cette intensité : FF = très forte, F = forte, mF = moyenne à forte, m = moyenne, mf = moyenne à faible, f = faible, ff = très faible.

Le tableau I représente le diagramme de diffraction des rayons X caractéristique des mordénites selon la demande de brevet français 89/13317. Dans la colonne des $d_{hkl}$ on a représenté les valeurs extrêmes que peuvent prendre les différentes distances interréticulaires $d_{hkl}$. Chacune de ces valeurs doit être affectée de l'erreur de mesure delta ($d_{hkl}$) généralement comprise entre ± 0,07 et ± 0,002 suivant la valeur de 2 thêta ($d_{hkl}$ est exprimée en Angström, 1 A = $10^{-10}$ m).

La mordénite brute de synthèse, synthétisée en milieu fluorure a été avantageusement préparée selon le procédé décrit et revendiqué dans la demande de brevet français 89/13317, procédé dont une description partielle est reprise ci-dessous à titre de référence :

a) on forme un mélange réactionnel en solution ayant un pH inférieur à environ 10, de préférence compris entre environ 4 et environ 10 (et de manière encore plus préférée compris entre environ 6 et environ 10), et comprenant de l'eau, au moins une source de silice, au moins une source d'aluminium, au moins une source d'agent mobilisateur contenant des ions fluorures (F⁻), une source de cations sodium (Na⁺), ledit mélange réactionnel ayant une composition, en termes de rapport molaire, comprise dans les intervalles de valeurs suivants :

Si/Al : 5-50, de préférence 5-25, par exemple 6,5-15,

F⁻/Si : 0,1-10, de préférence 0,25-8, par exemple 0,4-5,

Na⁺/Si : 0,1-10, de préférence 0,25-8, par exemple 0,4-5,

$H_2O$/Si : 5-25, de préférence 8-25, par exemple 8-22,

b) on maintient ledit mélange réactionnel à une température de chauffage comprise entre environ 90 et environ 250°C, de préférence entre environ 130 et environ 220°C, jusqu'à ce que l'on obtienne un composé cristallin.

Avant d'être utilisées comme bases catalytiques dans le procédé selon la présente invention, les mordénites brutes de synthèse doivent subir un certain nombre de modifications visant, notamment :

Tableau I

| 2 thêta (°) | $d_{hkl}$ (Å) | I/Io |
|:---:|:---:|:---:|
| 6,38 | 13,84 | mF |
| 8,50 | 10,39 | mf |
| 9,60 | 9,21 | F |
| 13,38 | 6,61 | mF |
| 13,70 | 6,46 | m |
| 14,35 | 6,17 | ff |
| 15,10 | 5,86 | f |
| 19,48 | 4,55 | mF |
| 20,72 | 4,28 | ff |
| 22,15 | 4,01 | F |
| 23,00 | 3,86 | ff |
| 23,50 | 3,78 | f |
| 24,30 | 3,66 | ff |
| 25,55 | 3,48 | FF |
| 26,20 | 3,398 | F |
| 27,65 | 3,223 | mF |
| 30,75 | 2,905 | m |
| 32,6 | 2,744 | ff |
| 33,08 | 2,706 | ff |
| 34,93 | 2,566 | ff |
| 35,55 | 2,523 | f |
| 40,39 | 2,231 | ff |
| 44,3 | 2,088 | f |

- à éliminer, si nécessaire, les composés organiques éventuellement présents dans le solide après la synthèse,
- à éliminer la majeure partie des cations sodium et à les remplacer par des protons,
- à optimiser les rapports atomiques Si/Al globaux et de charpente.

Pour atteindre ces objectifs, on peut utiliser toute technique connue de l'homme du métier :

. Pour éliminer les composés organiques éventuellement présents dans la mordénite après synthèse en milieu fluorure, on peut procéder à une calcination à une température d'au moins 350°C, de préférence d'au moins 450°C et par exemple comprise entre 500 et 600°C, pendant une durée d'au moins 10 minutes, de préférence comprise entre 30 et 180 minutes, en présence par exemple d'au moins 1 %, de préférence d'au moins 5 %, d'oxygène.

. Pour éliminer la majeure partie des cations sodium, on peut par exemple procéder à une ou plusieurs séries d'échanges dans des solutions de sel d'ammonium (chlorure, nitrate, sulfate etc) de concentration en ammonium généralement comprise entre 0,01 et 15N (ou dans des solutions d'acides divers (HCl, $H_2SO_4$, $HNO_3$) de normalité peu élevée), à une température comprise entre 10 et 180°C (échange sous pression autogène éventuellement), pendant une durée supérieure à 10 minutes environ.

. Pour obtenir les rapports atomiques Si/Al globaux et de charpente désirés, on peut recourir aux techniques de désalumination bien connues de l'homme du métier, telles que par exemple l'attaque acide directe de la forme NaM échangée partiellement ou non par $H^+$ ou $NH_4+$, la calcination en présence éventuellement de vapeur d'eau de la forme $NH_4M$ ou HM suivie, de préférence, d'un traitement chimique du type attaque acide (M = mordénite). L'attaque acide consiste en au moins un traitement dans des solutions d'acide de nature diverse (HCl, $HNO_3$, $H_2SO_4$, HF etc), à des températures comprises entre 10 et 150°C (attaque sous pression autogène éventuellement). Les concentrations en acide sont comprises entre 0,1 et 15N et, de préférence, entre 5 et 12N. Les rapports volume de solution sur poids de solide sec sont compris entre 3 et 20 $cm^3$/g et, de manière avantageuse, entre 3 et 7 $cm^3$/g. La durée du traitement est d'au moins 10 minutes. Pour atteindre les spécifications désirées, on peut avoir recours à un nombre limité d'attaques acides réalisées dans des conditions sévères ou à un nombre plus important d'attaques réalisées dans des conditions modérées. Le traitement acide direct sur la mordénite synthétisée en milieu fluorure peut également permettre d'éliminer la majeure partie des cations sodium et donc éviter l'étape préalable d'échange cationique. Le traitement thermique en présence de vapeur d'eau consiste habituellement en une calcination réalisée à une température supérieure à 350°C et, de préférence, supérieure à 500°C, pendant une durée d'au moins 10 minutes, sous une atmosphère contenant au moins 1 % de vapeur d'eau, de préférence au moins 10 % de vapeur d'eau. L'attaque acide qui suit éventuellement la calcination est réalisée dans les mêmes conditions que l'attaque acide décrite précédemment.

Le procédé de production d'un mélange de 2- et 3-phénylalcanes selon l'invention consiste à faire réagir, dans une zone de réaction, du benzène avec une charge renfermant au moins une oléfine linéaire comprenant de 9 à 16 atomes de carbone, de préférence de 10 à 14 atomes de carbone, dans sa molécule au contact d'au moins un catalyseur à base d'une mordénite, préparée à partir de la mordénite brute de synthèse synthétisée en milieu fluorure et décrite précédemment, ladite mordénite (à base de laquelle est ledit catalyseur) présentant les propriétés physico-chimiques suivantes :

- un rapport atomique Si/Al global compris entre 6 et 100, de préférence entre 15 et 60 et de manière encore plus préférée entre 20 et 50.
- une teneur pondérale en sodium inférieure à 1000 ppm, de préférence inférieure à 500 ppm,
- un volume de maille élémentaire inférieur à 2,781 $nm^3$ (l nm = $10^{-9}$m), de préférence inférieur à 2,748 $nm^3$,
- une fréquence de vibration asymétrique des tétraèdres $TO_4$ (T = Al ou Si) supérieure à 1076 $cm^{-1}$, de préférence supérieure à 1080 $cm^{-1}$, dans le spectre infra-rouge,
- un volume microporeux, mesuré par adsorption d'azote à 77 K sous une pression partielle d'azote de 0,19, supérieur à 0,160 $cm^3$ (liquide)/g, de préférence supérieur à 0,180 $cm^3$ (liquide)/g.

Sans nous lier à une théorie particulière, il est probable que les performances supérieures des mordénites employées dans l'invention résultent au moins en partie de la distribution des aluminium de charpente au sein des 4 sites cristallographiques non équivalents que comporte cette charpente. En effet, les propriétés catalytiques des zéolithes sont principalement liées à leurs propriétés acides. Celles-ci sont déterminées, outre par les rapports atomiques Si/Al de charpente et globaux, par la localisation exacte des aluminium de charpente. Dans la mordénite classique (synthèse en milieu $OH^-$), il est reconnu que les aluminium de charpente sont principalement localisés dans les cycles à 4 tétraèdres de la structure sous forme de paires Al-O-Si-Al (V. GRAMLICH, PhD Dissertation, ETH n° 4633, Zurich, 1971 ; G. DEBRAS, J.B. NAGY, Z. GABELICA, P. BODART, PA. JACOBS, Chem. Lett, 1983, p. 199). Cette configuration est par ailleurs favorisée thermodynamiquement (E.G. DEROUANE, J.G. FRIPIAT, Proc. 6th. Int. Zeolite. Conf, Reno, USA, 1984, p. 717). La présence de ces paires d'aluminium est favorable à des réactions bisites telles que les réactions de dismutation et de transalkylation, ainsi qu'aux réactions de cokage. Ce type de réaction est indésirable dans le cas de l'alkylation du benzène par les oléfines linéaires. En effet, on cherche à limiter le cokage de manière à assurer une durée de cycle maximale au catalyseur ; les réactions de dismutation et transalkylation sont par ailleurs à limiter le plus possible afin de maximiser la production de 2- et 3-phénylalcanes par passe. Dans les mordénites utilisées dans l'invention, la distribution des aluminium de charpente est différente ; ces paires aluminium sont certainement absentes ou tout du moins présentes en quantité très limitée, ce qui induit des propriétés acides et donc catalytiques différentes.

La mordénite définie précédemment peut être utilisée, dans la présente invention, seule ou en mélange avec un liant ou une matrice généralement choisis dans le groupe formé par les argiles, les alumines, la silice, la magnésie, la zircone, l'oxyde de titane, l'oxyde de bore et toute combinaison d'au moins deux des composés précités comme la silice-alumine, la silice-magnésie etc. Toutes les méthodes connues d'agglomération et de mise en forme sont applicables, telles que par exemple l'extrusion, le pastillage, la coagulation en goutte, le séchage par atomisation etc.

On utilise ainsi dans le procédé selon l'invention au moins un catalyseur à base d'une mordénite ayant les caractéristiques définies précédemment (notamment préparée à partir d'une mordénite synthétisée en milieu fluorure), contenant généralement 1 à 100 %, de préférence 20 à 98 % et, par exemple, 40 à 98 % en poids de ladite mordénite et 0 à 99 %, de préférence 2 à 80 % et, par exemple, 2 à 60 % en poids d'une matrice.

Selon une variante préférée du procédé de l'invention, on fait réagir, dans une zone de réaction, du benzène avec une charge renfermant au moins une oléfine linéaire comprenant de 9 à 16 atomes de carbone, de préférence de 10 à 14 atomes de carbone, dans sa molécule, au contact d'au moins un catalyseur à base d'une mordénite ayant les caractéristiques définies précédemment (réaction d'alkylation), puis on fractionne le produit obtenu de manière à recueillir séparément une première fraction renfermant du benzène non-converti, une deuxième fraction renfermant au moins une oléfine linéaire $C_9$-$C_{16}$ (de préférence $C_{10}$-$C_{14}$) non-convertie, une troisième fraction renfermant des 2- et 3-phénylalcanes et une quatrième fraction renfermant au moins un poly-alkylbenzène (ou fraction poly-alkylbenzène), celle-ci étant ensuite, au moins en partie, recyclée vers ladite zone de réaction où elle réagit donc avec du benzène au contact dudit catalyseur, c'est-à-dire du catalyseur mentionné ci-dessus à base de la mordénite définie précédemment, afin d'être au moins en partie transalkylée (réaction de transalkylation), et on recueille un mélange de 2- et 3-phénylalcanes.

Cette variante de l'invention est donc notamment caractérisée par le fait que les réactions d'alkylation et de transalkylation ont lieu conjointement dans la même zone de réaction (c'est-à-dire dans le même réacteur) en présence du même catalyseur.

De manière préférée, la première fraction renfermant du benzène non-converti à l'issue de la réaction d'alkylation est au moins en partie recyclée vers ladite zone de réaction : ainsi, au moins une partie du benzène réagissant avec au moins une partie de la quatrième fraction (ou fraction poly-alkylbenzène) recyclée est constituée de benzène non-converti lors de la réaction d'alkylation, donc de benzène non-converti provenant de ladite première fraction.

De même, de manière préférée, la deuxième fraction renfermant au moins une oléfine linéaire $C_9$-$C_{16}$ (habituellement $C_{10}$-$C_{14}$) non-convertie à l'issue de la réaction d'alkylation est au moins en partie recyclée vers ladite zone de réaction.

La partie recyclée de la quatrième fraction contient de préférence essentiellement au moins un dialkylbenzène et est de préférence sensiblement exempte d'alkyl-aromatiques lourds qui peuvent être éliminés par fractionnement éventuellement.

Le mélange de 2-phénylalcanes et 3-phénylalcanes obtenu selon l'invention est de manière avantageuse quasi-exempt ou sensiblement exempt de n-phénylalcanes, n étant un nombre entier supérieur ou égal à 4.

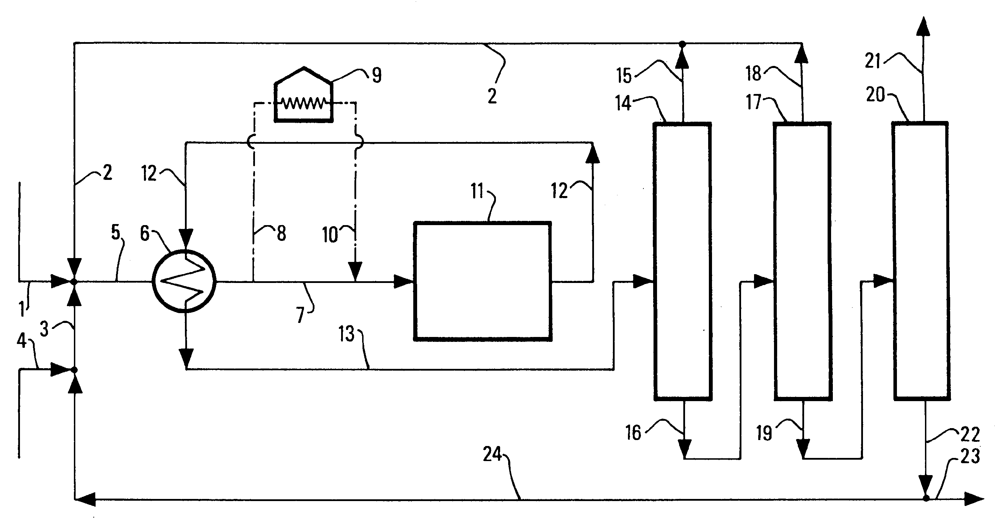

La figure unique illustre un mode particulier de réalisation de l'invention.

Du benzène frais (conduite 1) est mélangé, d'une part, avec un mélange (conduite 2) de benzène et d'oléfines linéaires $C_{10}$-$C_{14}$ (coupe dont l'intervalle de distillation se situe entre 165 et 240°C), le benzène provenant, par la conduite 15, de la tête d'une première colonne de fractionnement 14 et lesdites oléfines $C_{10}$-$C_{14}$ provenant, par la conduite 18, de la tête d'une deuxième colonne de fractionnement 17, et, d'autre part, avec un mélange (conduite 3) d'oléfines linéaires "fraîches" $C_{10}$-$C_{14}$ arrivant par la conduite 4 et de dialkylbenzènes provenant, par la conduite 24, du fond (conduite 22) d'une troisième colonne de fractionnement 20, après une purge éventuelle (conduite 23). Le mélange global (ou charge) obtenu est ensuite envoyé, par la conduite 5, dans l'échangeur de chaleur 6 où il est préchauffé par échange de chaleur avec l'effluent issu, par la conduite 12, du réacteur d'alkylation 11. Puis le mélange est envoyé, après son séjour dans l'échangeur de chaleur 6, dans le réacteur d'alkylation 11 par la conduite 7. Eventuellement, si le préchauffage dans l'échangeur de chaleur 6 est insuffisant, le mélange sortant de cet échangeur 6 est préalablement envoyé, par la conduite 8, dans le four de chauffage 9, d'où il ressort par la conduite 10 pour se diriger vers le réacteur d'alkylation 11. A la sortie du réacteur 11, l'effluent est envoyé, par la conduite 12, vers l'échangeur de chaleur 6, puis, par la conduite 13, vers une première colonne de fractionnement 14. En tête de cette première colonne de fractionnement 14, on sort, par la conduite 15, le benzène en excès qui n'a pas réagi et que l'on recycle ensuite, après mélange avec les oléfines linéaires $C_{10}$-$C_{14}$ non-transformées provenant, par la conduite 18, de la tête d'une deuxième colonne de fractionnement 17, vers l'entrée du réacteur 11 par la conduite 2. En fond de cette première colonne de fractionnement 14, on recueille un mélange qui est envoyé, par la conduite 16, vers une deuxième colonne de fractionnement 17. En tête de cette deuxième colonne de fractionnement 17, on recueille, par la conduite 18, les oléfines linéaires $C_{10}$-$C_{14}$ non-transformées que l'on recycle ensuite, après mélange avec le benzène provenant, par la conduite 15, de la tête de la première colonne de fractionnement 14, vers l'entrée du réacteur

6

11 par la conduite 2. En fond de cette deuxième colonne de fractionnement 17, on soutire un mélange qui est envoyé, par la conduite 19, vers une troisième colonne de fractionnement 20. En tête de cette troisième colonne de fractionnement 20, on recueille, par la conduite 21, un mélange de 2-phénylalcanes et 3-phénylalcanes (coupe dont l'intervalle de distillation est situé entre 290 et 370°C) qui est envoyé au stockage. En fond de cette troisième colonne de fractionnement 20, on soutire, par la conduite 22, des dialkylbenzènes dont au moins une grande partie est recyclée, par la conduite 24, vers le réacteur 11 et dont une faible quantité est éventuellement purgée du circuit par la conduite 23.

Le procédé selon la présente invention peut être effectué à une température inférieure à 400°C, de préférence inférieure à 60°C, sous une pression de 1 à 10 MPa, avec un débit d'hydrocarbures liquides (vitesse spatiale) d'environ 0,5 à 50 volumes par volume de catalyseur et par heure et avec un rapport molaire benzène/oléfine(s) linéaire(s) $C_9$-$C_{16}$ compris entre 1 et 20.

EXEMPLE 1 Préparation de trois catalyseurs A, B, C.

La matière première utilisée est une mordénite (exempte de composés organiques) synthétisée en milieu fluorure selon le procédé de préparation décrit dans la demande de brevet français 89/13317.

100 grammes de cette mordénite forme sodique sont partagés en trois parties égales $A_o$, $B_o$ et $C_o$ qui sont chacune soumises à une attaque acide à l'aide de solutions aqueuses d'acide nitrique respectivement de normalité 4,5 N, 10 N et 14 N pendant une durée de 4 heures pour l'attaque acide 4,5 N et de 6 heures pour les attaques acides 10 et 14 N : chaque solide est ainsi porté à reflux dans la solution aqueuse d'acide nitrique, avec un rapport volume de solution aqueuse d'acide nitrique sur poids de mordénite sèche (V/P) respectivement de 5,4 et 5 cm³/g, puis lavé à l'eau.

Les caractéristiques des mordénites obtenues $A_1$, $B_1$ et $C_1$ figurent dans le tableau II.

Chaque mordénite $A_1$, $B_1$ et $C_1$ préparée précédemment est ensuite mélangée avec du gel d'alumine. Chaque mélange obtenu est ensuite mis sous forme d'extrudés de diamètre égal à environ 1,8 mm

Tableau II

| | $A_1$ | $B_1$ | $C_1$ |
|---|---|---|---|
| Na (ppm poids) | 200 | 50 | 10 |
| Si/Al (atomique global) | 10 | 45 | 98 |
| Volume de maille élémentaire (nm³) | < 2,733 | < 2,733 | < 2,733 |
| Fréquence infra-rouge $TO_4$ (cm$^{-1}$) | >1080 | >1080 | > 1080 |
| Volume microporeux (cm³/g) (adsorption d'azote) | > 0,160 | > 0,160 | >0,160 |

par passage au travers d'une filière. Les extrudés sont ensuite séchés dans une étuve à 120°C pendant une nuit, puis calcinés sous air sec jusqu'à 550°C environ.

On obtient alors des catalyseurs A, B et C à base respectivement des mordénites $A_1$, $B_1$ et $C_1$, contenant 80 % en poids de mordénite et 20 % en poids d'alumine.

## EXEMPLE 2

Les trois catalyseurs A, B et C, préparés dans l'exemple 1, sont chacun testés en alkylation du benzène par le dodécène-1, pour la production de 2- et 3-phényldodécanes, dans les conditions opératoires suivantes :
- température : 50°C,
- pression : 4 MPa,
- débit horaire de charge liquide égal à 3 fois le volume du catalyseur,
- rapport molaire benzène/dodécène-1 : 5,5.

Les résultats obtenus sont présentés dans le tableau III.

## EXEMPLE 3 Préparation de trois catalyseurs D, E, F.

On prépare ici trois catalyseurs D, E, F à base de mordénite synthétisée en milieu OH⁻.

La matière première utilisée est une mordénite larges pores de la société Tosoh, sous forme sodique, référencée TSZ 600 NAA. Sa formule chimique, à l'état anhydre, est $Na, AlO_2 (SiO_2)_{5,1}$ et sa teneur en sodium est de l'ordre de 5 % en poids.

100 grammes de cette poudre sont portés à reflux à 100°C pendant 2 heures dans une solution de nitrate d'ammonium 4 M ; le volume de la solution de nitrate d'ammonium engagée est égal à 4 fois le poids de mordénite sèche (V/P = 4 cm³/g). Cette opération d'échange cationique est recommencée 3 fois. La teneur pondérale en sodium du produit obtenu est d'environ 500 ppm.

Ce produit est ensuite séparé en trois parties égales $D_o$, $E_o$ et $F_o$ qui sont chacune soumises à une attaque acide à l'aide de solutions aqueuses d'acide nitrique respectivement de normalité 0,9 N, 4,5 N et 8 N : chaque solide est ainsi porté à reflux dans la solution

## Tableau III

| CATALYSEUR | A | B | C |
|---|---|---|---|
| **COMPOSITION DE LA CHARGE** (% poids) | | | |
| Benzène | 71,7 | 71,7 | 71,7 |
| Dodécène-1 | 28,3 | 28,3 | 28,3 |
| **COMPOSITION DU PRODUIT OBTENU** (% poids) | | | |
| Benzène | 62,1 | 59 | 59,6 |
| Dodécène-1 | - | - | 0,6 |
| Isomères du dodécène-1 | 7 | - | - |
| 2-phényldodécane | 18,8 | 34,1 | 31,6 |
| 3-phényldodécane | 7,4 | 4,4 | 4 |
| 4-phényldodécane | 2,1 | 0,3 | 0,3 |
| 5-phényldodécane | 0,8 | - | - |
| 6-phényldodécane | 0,4 | - | - |
| Didodécylbenzènes | 1,3 | 2 | 3,5 |
| Résidu lourd | 0,1 | 0,2 | 0,4 |
| **TAUX DE CONVERSION PAR PASSE** | | | |
| Benzène | 13,4 % | 17,7 % | 16,9 % |
| Dodécène-1 | 100 % | 100 % | 97,8 % |
| **SELECTIVITES PAR RAPPORT AU DODECENE-1 TRANSFORME** | | | |
| 2-phényldodécane | 45,4 % | 82,4 % | 77,9 % |
| 3-phényldodécane | 17,9 % | 10,5 % | 9,9 % |
| Total | 63,3 % | 92,9 % | 87,8 % |

aqueuse d'acide nitrique, avec un rapport V/P égal a 4 cm³/g, puis lavé à l'eau.

Les caractéristiques des mordénites obtenues $D_1$, $E_1$ et $F_1$ figurent dans le tableau IV.

TABLEAU IV

| | $D_1$ | $E_1$ | $F_1$ |
|---|---|---|---|
| Na (ppm poids) | 185 | 25 | 10 |
| Si/Al (atomique global) | 13 | 40 | 90 |
| Volume de maille élémentaire $(nm^3)$ | 2,747 | 2,715 | 2,690 |
| Fréquence infra-rouge $TO_4$ $(cm^{-1})$ | 1080,2 | 1086,0 | 1091,7 |
| Volume microporeux $(cm^3/g)$ (adsorption d'azote) | > 0,160 | > 0,160 | > 0,160 |

Chaque mordénite $D_1$, $E_1$ et $F_1$ préparée précédemment est ensuite mélangée avec du gel d'alumine. Chaque mélange obtenu est ensuite mis sous forme d'extrudés de diamètre égal à environ 1,8 mm par passage au travers d'une filière. Les extrudés sont ensuite séchés dans une étuve à 120°C pendant une nuit, puis calcinés sous air sec jusqu'à 550°C environ.

On obtient alors des catalyseurs D, E et F à base respectivement des mordénites $D_1$, $E_1$ et $F_1$, contenant 80 % en poids de mordénite et 20 % en poids d'alumine.

EXEMPLE 4

Les trois catalyseurs D, E et F, préparés dans l'exemple 3, sont chacun testés en alkylation du benzène par le dodécène-1, pour la production de 2- et 3-phényldodécanes, dans des conditions opératoires identiques à celles utilisées dans l'exemple 2.

Les résultats obtenus sont présentés dans le tableau V.

En comparant les résultats figurant dans les tableaux III et V, on peut constater qu'il est préférable de travailler avec les catalyseurs préconisés par l'invention, c'est-à-dire qu'il faut utiliser des mordénites préparées à partir de mordénites synthétisées en milieu fluorure : en effet, pour des rapports atomiques Si/Al globaux voisins (cas de A et D, de B et E, de C et F), on obtient avec les catalyseurs préconisés par l'invention (A, B et C) une sélectivité en 2- et 3-phényldodécanes très supérieure (6 à 8 points) à celle obtenue avec les catalyseurs à base de mordénite synthétisée en milieu $OH^-$ (D, E et F) ; de plus, pour des rapports atomiques Si/Al globaux voisins, l'activité des catalyseurs A, B et C apparaît également meilleure que celle des catalyseurs D, E et F.

Enfin, la lecture des résultats présentés dans le tableau III montre que, parmi les catalyseurs préconisés par l'invention, il est avantageux de travailler avec un catalyseur à base d'une mordénite de rapport atomique Si/Al global compris entre 15 et 60 (cas du catalyseur B) qui présente une activité et surtout une sélectivité en 2- et 3-phényldodécanes encore meilleure.

EXEMPLE 5

En opérant selon le procédé de l'invention conforme à la figure unique, c'est-à-dire notamment en recyclant les didodécylbenzènes et le benzène vers le réacteur d'alkylation contenant le catalyseur B préparé dans l'exemple 1, on obtient une sélectivité totale en 2- et 3-phényldodécanes par rapport au

Tableau V

| CATALYSEUR | D | E | F |
|---|---|---|---|
| COMPOSITION DE LA CHARGE (% poids) | | | |
| Benzène | 71,7 | 71,7 | 71,7 |
| Dodécène-1 | 28,3 | 28,3 | 28,3 |
| COMPOSITION DU PRODUIT OBTENU (% poids) | | | |
| Benzène | 62,6 | 59,4 | 61,3 |
| Dodécène-1 | - | - | 3,1 |
| Isomères du dodécène-1 | 7,9 | - | - |
| 2-phényldodécane | 14,4 | 31,4 | 26,6 |
| 3-phényldodécane | 8,6 | 4,4 | 3,3 |
| 4-phényldodécane | 2,7 | 0,4 | 0,4 |
| 5-phényldodécane | 1,3 | - | - |
| 6-phényldodécane | 0,5 | - | - |
| Didodécylbenzènes | 1,8 | 4,1 | 4,4 |
| Résidu lourd | 0,2 | 0,3 | 0,9 |
| TAUX DE CONVERSION PAR PASSE | | | |
| Benzène | 12,7 % | 17,2 % | 14,5 % |
| Dodécène-1 | 100 % | 100 % | 89 % |
| SELECTIVITES PAR RAPPORT AU DODECENE-1 TRANSFORME | | | |
| 2-phényldodécane | 34,7 % | 75,8 % | 72,1 % |
| 3-phényldodécane | 20,8 % | 10,6 % | 8,9 % |
| Total | 55,5 % | 86,4 % | 81 % |

dodécène-1 transformé de 98,5 %. Ainsi, on obtient donc, avec une sélectivité très élevée, un mélange de 2-

EP 0 469 940 B1

et 3-phényldodécanes conduisant à des détergents de très grande qualité et parfaitement biodégradables.

**Revendications**

1. Procédé de production d'un mélange de 2-phénylalcanes et 3-phénylalcanes dans lequel on fait réagir, dans une zone de réaction, du benzène avec une charge renfermant au moins une oléfine linéaire comprenant de 9 à 16 atomes de carbone dans sa molécule au contact d'au moins un catalyseur à base d'une mordénite, ledit procédé étant caractérisé:
   - PREMIÈREMENT en ce que l'on fractionne ensuite le produit obtenu de manière à recueillir séparément une première fraction renfermant du benzène non-converti, une deuxième fraction renfermant au moins une oléfine linéaire, comportant de 9 à 16 atomes de carbone dans sa molécule, non-convertie, une troisième fraction renfermant un mélange de 2-phénylalcanes et 3-phénylalcanes et une quatrième fraction renfermant au moins un poly-alkylbenzène, ladite quatrième fraction étant ensuite, au moins en partie, recyclée vers ladite zone de réaction où elle réagit avec du benzène en présence dudit catalyseur afin d'être au moins en partie transalkylée, et on recueille un mélange de 2-phénylalcanes et 3-phénylalcanes.
   - deuxièmement en ce que ladite mordénite a un rapport atomique Si/Al global compris entre 6 et 100, une teneur pondérale en sodium inférieure à 1 000 ppm, un volume de maille élémentaire inférieur à 2,781 nm$^3$, une fréquence de vibration asymétrique des tétraèdres $TO_4$ (T = Al ou Si) supérieure à 1076 cm$^{-1}$ dans le spectre infra-rouge et un volume microporeux, mesuré par adsorption d'azote à 77 K sous une pression partielle d'azote de 0,19, supérieur à 0,160 cm$^3$ (liquide)/g, ladite mordénite ayant été en outre préparée à partir d'une mordénite synthétisée en milieu fluorure qui possède
     a) la formule générale approchée suivante :
     $$Na_2O, Al_2O_3, xSiO_2$$
     où x est un nombre compris entre 9 et 30, et
     b) un diagramme de diffraction des rayons X représenté ci-après :

| 2 thêta (°) | $d_{hkl}$ (Å) | I/Io |
|:---:|:---:|:---:|
| 6,38 | 13,84 | mF |
| 8,50 | 10,39 | mf |
| 9,60 | 9,21 | F |
| 13,38 | 6,61 | mF |
| 13,70 | 6,46 | m |
| 14,35 | 6,17 | ff |
| 15,10 | 5,86 | f |
| 19,48 | 4,55 | mF |
| 20,72 | 4,28 | ff |
| 22,15 | 4,01 | F |
| 23,00 | 3,86 | ff |
| 23,50 | 3,78 | f |
| 24,30 | 3,66 | ff |
| 25,55 | 3,48 | FF |
| 26,20 | 3,398 | F |
| 27,65 | 3,223 | mF |
| 30,75 | 2,905 | m |
| 32,6 | 2,744 | ff |
| 33,08 | 2,706 | ff |
| 34,93 | 2,566 | ff |
| 35,55 | 2,523 | f |
| 40,39 | 2,231 | ff |
| 44,3 | 2,088 | f |

et troisièmement par une température opératoire inférieure à 60° C.

2. Procédé selon la revendication 1 dans lequel ladite mordénite a un rapport atomique Si/Al global compris entre 15 et 60.

3. Procédé selon l'une des revendications 1 et 2 dans lequel ladite mordénite est telle que :
   - son rapport atomique Si/Al global est compris entre 20 et 50 ;
   - sa teneur pondérale en sodium est inférieure à 500 ppm ;
   - son volume de maille élémentaire est inférieur à 2,748 nm$^3$ ;
   - la fréquence de vibration asymétrique des tétraèdres $TO_4$ (T = Al ou Si) est supérieure à 1080 cm$^{-1}$ dans le spectre infrarouge ;
   - son volume microporeux, mesuré par adsorption d'azote à 77 K sous une pression partielle d'azote de 0,19, est supérieur à 0,180 cm$^3$ (liquide)/g.

4. Procédé selon l'une des revendications 1 à 3 dans lequel ladite première fraction est au moins en partie

recyclée vers ladite zone de réaction.

5. Procédé selon l'une des revendications 1 à 4 dans lequel ladite deuxième fraction est au moins en partie recyclée vers ladite zone de réaction.

6. Procédé selon l'une des revendications 1 à 5 dans lequel ledit catalyseur contient en outre une matrice choisie dans le groupe formé par les argiles, les alumines, la silice, la magnésie, la zircone, l'oxyde de titane, l'oxyde de bore et toute combinaison d'au moins deux des composés précités.

**Patentansprüche**

1. Verfahren zur Herstellung von 2-Phenylalkanen und 3-Phenylalkanen, bei dem man in einer Reaktionszone Benzol mit einem Reaktionseintrag, der mindestens ein lineares Olefin, das 9 bis 16 Kohlenstoffatome in seinem Molekül besitzt, im Kontakt mit mindestens einem Katalysator auf der Grundlage von einem Mordenit reagieren läßt, wobei das besagte Verfahren gekennzeichnet ist,
dadurch, daß anschließend das gewonnene Produkt so fraktioniert wird, daß getrennt eine erste Fraktion, die nicht umgesetzes Benzol umfaßt, eine zweite Fraktion, die mindestens ein nicht umgesetztes lineares Olefin mit 9 bis 16 Kohlenstoffatomen in seinem Molekül umfaßt, eine dritte Fraktion, die eine Mischung aus 2-Phenylalkanen und 3-Phenylalkanen umfaßt und eine vierte Fraktion, die mindestens ein Polyalkylbenzol umfaßt, gewonnen werden, wobei die besagte vierte Fraktion zumindest zum Teil in die besagte Reaktionszone zurückgeführt wird, wo sie mit Benzol in Gegenwart des besagten Katalysators in der Weise reagiert, daß sie mindestens zum Teil transalkyliert wird und man eine Mischung aus 2-Phenylalkanen und 3-Phenylalkanen gewinnt, und
dadurch, daß der besagte Mordenit ein Gesamtatomverhältnis Si/Al zwischen einschließlich 6 und 100 hat, einen Gewichtsanteil an Natrium von weniger als 1000 ppm, ein Volumen der Elementarzelle, das kleiner als 2,781 nm³ ist, eine Frequenz der asymmetrischen Schwingung der $TO_4$-Tetraeder (T = Al oder Si) im Infrarot-Spektrum von höher als 1076 cm⁻¹ und ein Mikroporenvolumen, gemessen durch Adsorption von Stickstoff bei 77 K unter einen Stickstoffpartialdruck von 0,19, das größer als 0,160 cm³ (Flüssigkeit)/g ist, wobei der besagte Mordenit darüber hinaus von einem in fluorhaltigem Milieu synthetisierten Mordenit ausgehend, hergestellt wurde, der
a) die folgende allgemeine Näherungsformel besitzt:
$$Na_2O,Al_2O_3 \ x \ SiO_2$$
in der x eine Zahl zwischen einschließlich 9 und 30 ist, und
b) ein Röntgendiffraktogramm besitzt, das im folgenden dargestellt ist:

| 2θ (°) | $d_{hkl}$ (Å) | $I/I_0$ |
|---|---|---|
| 6,38 | 13,84 | mF |
| 8,50 | 10,39 | mf |
| 9,60 | 9,21 | F |
| 13,38 | 6,61 | mF |
| 13,70 | 6,46 | m |
| 14,35 | 6,17 | ff |
| 15,10 | 5,86 | f |
| 19,48 | 4,55 | mF |
| 20,72 | 4,28 | ff |
| 22,15 | 4,01 | F |
| 23,00 | 3,86 | ff |
| 23,50 | 3,78 | f |
| 24,30 | 3,66 | ff |
| 25,55 | 3,48 | FF |
| 26,20 | 3,398 | F |
| 27,65 | 3,223 | mF |
| 30,75 | 2,905 | m |
| 32,6 | 2,744 | ff |
| 33,08 | 2,706 | ff |
| 34,93 | 2,566 | ff |
| 35,55 | 2,523 | f |
| 40,39 | 2,231 | ff |
| 44,3 | 2,088 | f |

und das besagte Verfahren darüber hinaus durch eine Arbeitstemperatur von weniger als 60 °C gekennzeichnet ist.

2. Verfahren nach Anspruch 1, bei dem der besagte Mordenit ein Gesamtatomverhältnis Si/Al zwischen einschließlich 15 und 60 hat.

3. Verfahren nach einem der Ansprüche 1 und 2, bei dem der besagte Mordenit
   - ein Gesamtatomverhätnis Si/Al zwischen einschließlich 20 und 50 aufweist;
   - sein Gewichtsanteil an Natrium unterhalb von 500 ppm liegt;
   - sein Volumen der Elementarzelle kleiner als 2,748 $nm^3$ ist;
   - die Frequenz der asymmetrischen Schwingung der $TO_4$-Tetraeder (T = Al oder Si) im Infrarot-Spektrum von höher als 1080 $cm^{-1}$ liegt;
   - sein Mikroporenvolumen, gemessen durch Adsorption von Stickstoff bei 77 K unter einen Stickstoffpartialdruck von 0,19, größer als 0,180 $cm^3$ (Flüssigkeit)/g ist.

4. Verfahren nach einem der Ansprüche 1 bis 3, bei dem die besagte erste Fraktion zumindest teilweise zu der besagten Reaktionszone zurückgeführt wird.

5. Verfahren nach einem der Ansprüche 1 bis 4, bei dem die besagte zweite Fraktion zumindest teilweise zu der besagten Reaktionszone zurückgeführt wird.

6. Verfahren nach einem der Ansprüche 1 bis 5, bei dem der besagte Katalysator darüber hinaus eine Matrix umfaßt, die aus der von den Tonen, den Aluminaten, Silicat, Magnesiumoxid, Zirkon, Titanoxid, Boroxid oder jeder Kombination von mindestens zwei der vorgenannten Verbindungen gebildeten Gruppe gewählt wird.

**Claims**

1. Process for the production of a mixture of 2-phenyl alkanes and 3-phenyl alkanes, where reaction takes place in a reaction zone of the benzene with a charge containing at least one linear olefin incorporating 9 to 16 carbon atoms in its molecule in contact with at least one mordenite-based catalyst, said process being characterized in that firstly fractionation then takes place of the product obtained so as to separately collect a first fraction containing unconverted benzene, a second fraction containing at least one linear olefin having 9 to 16 carbon atoms in its molecule and in the unconverted state, a third fraction containing a mixture of 2-phenyl alkanes and 3-phenyl alkanes and a fourth fraction containing at least one polyalkyl benzene, said fourth fraction then being at least partly recycled to said reaction zone, where it reacts with benzene in the presence of the catalyst in order to at least partly be transalkylated and a mixture of 2-phenyl alkanes and 3-phenyl alkanes is collected and secondly in that said mordenite has a total Si/Al atomic ratio between 6 and 100, a sodium weight content below 1000 ppm, a unit mesh volume below 2.781 nm$^3$, an asymmetrical vibration frequency of the tetrahedrons $TO_4$ (T = Al or Si) above 1076 cm$^{-1}$ in the infrared spectrum and a micropore volume, measured by nitrogen adsorption at 77 K under a partial nitrogen pressure of 0.19, higher than 0.160 cm$^3$ (liquid)/g, said mordenite having been prepared from a mordenite synthesized in the fluoride medium and which has

   a) the following approximate general formula:

   $$Na_2O, Al_2O_3, xSiO_2$$

   in which x is a number between 9 and 30 and

   b) a X-ray diffraction diagram represented hereinafter:

| 2 theta ($^{\circ}$) | $d_{hkl}$ ($\AA$) | I/Io |
|---|---|---|
| 6.38 | 13.84 | mF |
| 8.50 | 10.39 | mf |
| 9.60 | 9.21 | F |
| 13.38 | 6.61 | mF |
| 13.70 | 6.46 | m |
| 14.35 | 6.17 | ff |
| 15.10 | 5.86 | f |
| 19.48 | 4.55 | mF |
| 20.72 | 4.28 | ff |
| 22.15 | 4.01 | F |
| 23.00 | 3.86 | ff |
| 23.50 | 3.78 | f |
| 24.30 | 3.66 | ff |
| 25.55 | 3.48 | FF |
| 26.20 | 3.398 | F |
| 27.65 | 3.223 | mF |
| 30.75 | 2.905 | m |
| 32.6 | 2.744 | ff |
| 33.08 | 2.706 | ff |
| 34.93 | 2.566 | ff |
| 35.55 | 2.523 | f |
| 40.39 | 2.231 | ff |
| 44.3 | 2.088 | f |

and thirdly by an operating temperature below 60°C.

2. Process according to claim 1, wherein said mordenite has a total Si/Al atomic ratio between 15 and 60.

3. Process according to either of the claims 1 and 2, wherein said mordenite is such that its total Si/Al atomic ratio is between 20 and 50, its sodium weight content is below 500 ppm, its unit mesh volume is below 2.748 nm$^3$, the asymmetrical vibration frequency of the tetrahedrons $TO_4$ (T = Al or Si) is above 1080 cm$^{-1}$ in the infrared spectrum and its micropore volume measured by nitrogen adsorption at 77 K under a partial nitrogen pressure of 0.19, exceeds 0.180 cm$^3$ (liquid)/g.

4. Process according to any one of the claims 1 to 3, wherein said first fraction is at least partly recycled to said reaction zone.

5. Process according to any one of the claims 1 to 4, wherein said second fraction is at least partly recycled to said reaction zone.

6. Process according to any one of the claims 1 to 5, wherein said catalyst also contains a matrix chosen from within the group formed by clays, aluminas, silica, magnesia, zirconia, titanium dioxide, boric oxide and any combination of at least two of the aforementioned compounds.

EP 0 469 940 B1